Europäisches Patentamt

European Patent Office

Office européen des brevets

(11) Numéro de publication: **0 467 743 B1**

(12) **FASCICULE DE BREVET EUROPEEN**

(45) Date de publication de fascicule du brevet: **30.11.94**  (51) Int. Cl.⁵: **B29C 43/00**, B29C 67/24, A61K 7/035

(21) Numéro de dépôt: **91401870.0**

(22) Date de dépôt: **05.07.91**

(54) Procédé de compactage d'un mélange pulvérulent permettant d'obtenir un produit compact absorbant ou partiellement délitable et produit obtenu par ce procédé.

(30) Priorité: **20.07.90 FR 9009327**

(43) Date de publication de la demande:
**22.01.92 Bulletin 92/04**

(45) Mention de la délivrance du brevet:
**30.11.94 Bulletin 94/48**

(84) Etats contractants désignés:
**DE ES FR GB IT**

(56) Documents cités:
DE-A- 3 437 989
FR-A- 2 109 838
GB-A- 877 528
US-A- 3 717 427
US-A- 4 597 922

PATENT ABSTRACTS OF JAPAN, vol. 7, no.
95 (M-209)[1240], 21 avril 1983;& JP-A-58 20
413 (AKEBONO BRAKE KOGYO) 05-02-1983

(73) Titulaire: **L'OREAL**
**14, Rue Royale**
**F-75008 Paris (FR)**

(72) Inventeur: **Gueret, Jean-Louis**
**15, rue Hégésippe-Moreau**
**F-75018 Paris (FR)**

(74) Mandataire: **Peuscet, Jacques et al**
**Cabinet Peuscet**
**68, rue d'Hauteville**
**F-75010 Paris (FR)**

Rank Xerox (UK) Business Services
(3.10/3.09/3.3.3)

**EP 0 467 743 B1**

**Description**

La présente invention concerne un procédé de compactage d'un mélange pulvérulent permettant d'obtenir un produit compacté absorbant ou partiellement délitable et le produit compacté obtenu.

DE-C-34 37 989 décrit un procédé de fabrication de mines pour l'écriture, par exemple de mines de crayon, comportant des pigments maintenus par un liant constitué par un produit thermoplastique. Une poudre comportant la poudre de pigment, le liant ainsi que divers additifs est versée dans une douille et subit d'abord une légère compression, par exemple de 0,5 à 2 bars (voir colonne 5, lignes 38 et suivantes). La douille, ainsi remplie de la poudre compactée, est ensuite introduite dans un four et chauffée à une température telle qu'une fusion des particules de liant se produit dans des zones limitées des particules de pigment.

On évite, ainsi, d'enrober totalement les particules de pigment par du liant.

Ce procédé, destiné à des mines de crayon, ou analogues, est relativement long à mettre en oeuvre. En outre, il n'est pas approprié à la préparation de produits plats tels que des pastilles ou des comprimés obtenus à partir de produits pharmaceutiques, ou des "compacts" de produits cosmétiques pour le maquillage du visage (fard à joues, fard à paupières).

Pour effectuer le compactage de tels comprimés, pastilles, ou "compacts", on dispose le mélange pulvérulent à compacter dans le fond d'une enceinte, dans laquelle on fait coulisser un piston ou poinçon, qui vient comprimer le mélange pulvérulent pour assurer la cohésion des grains du (des) produit(s) pulvérulent(s).

Dans le cas des produits cosmétiques, le compactage doit permettre un délitage ultérieur par frottement et, dans ces conditions, la pastille comprimée obtenue est extrêmement fragile, surtout en faible épaisseur ; on est donc amené à la fabriquer dans une coupelle pour permettre la manipulation après compactage. Le fond de l'enceinte de compactage est donc constitué par une coupelle en métal ou en matière plastique, dans laquelle on fabrique un produit compacté généralement appelé "compact" ; on utilise ensuite le "compact" obtenu dans sa coupelle. Jusqu'à présent, on ne savait pas obtenir un "compact" qui soit à la fois suffisamment délitable pour que le fard puisse être prélevé et assez solide pour pouvoir être manipulé sans coupelle.

D'autre part, il est connu que les procédés de compactage nécessitent l'emploi de pressions élevées qui varient selon la poudre à compacter. Pour abaisser la pression nécessaire pour effectuer le compactage et faciliter la cohésion des grains du produit pulvérulent, on ajoute généralement un liant, le plus connu étant le stéarate de zinc.

La présente invention a pour objet un procédé qui permet de préparer des "compacts" à la fois délitables et solides sans utilisation de coupelle et qui permet d'utiliser des pressions de compactage plus faibles.

La présente invention concerne un procédé de compactage d'un mélange pulvérulent permettant d'obtenir un produit compacté absorbant ou partiellement délitable, dans lequel on dépose une quantité de mélange pulvérulent au fond d'une enceinte et on fait coulisser dans l'enceinte un piston qui exerce une pression de compactage sur le mélange pulvérulent, lequel contient au moins un produit thermoplastique, le reste étant constitué par au moins un produit non-thermoplastique, caractérisé par le fait que le mélange pulvérulent contient de 5 à 80 % en poids de produit thermoplastique et que pendant que l'on exerce la pression, on soumet le mélange pulvérulent à l'action d'ultra-sons.

Dans la définition ci-dessus et dans tout le texte de la présente demande, le terme "produit" qui est utilisé peut désigner soit un composé unique soit un mélange de plusieurs composés différents.

On a trouvé que lorsqu'on effectue le compactage sous ultra-sons, la présence d'un produit thermoplastique dans le mélange pulvérulent permet de créer dans le produit pulvérulent une armature interne et/ou externe (comme il sera expliqué plus en détail par la suite), qui maintient le produit non-thermoplastique et permet d'obtenir des compacts pouvant absorber des liquides, lorsque le produit non-thermoplastique compacté contient des produits absorbants, ou des compacts partiellement délitables. Lorsque la quantité de produit thermoplastique est inférieure à 5 % en poids, il n'y a plus formation d'une armature suffisante maintenant le produit non-thermoplastique et lorsque la quantité de produit thermoplastique est supérieure à 80 % en poids, on ne peut obtenir un produit compacté, qui reste suffisamment absorbant ou délitable.

Les produits thermoplastiques utilisables comprennent tout polymère, copolymère ou tout mélange de polymères et/ou copolymères ayant la propriété d'être thermoplastique dans les conditions opératoires. Comme polymère thermoplastique utilisable selon l'invention, on peut citer le polyéthylène, le polystyrène, les polyamides, le chlorure de polyvinyle, et le polyéthylène téréphtalate. Dans le cas où on désire un produit compacté parfumé, on peut utiliser un polyéthylène parfumé, par exemple celui vendu sous la marque "ENKA FRAGRANCE CARRIERS" par la société "ENKA AG."

2

On utilise, de préférence, un produit thermoplastique ayant une granulométrie, homogène ou non, comprise entre 5 et 500 μ.

Les produits non-thermoplastiques utilisables comprennent tout produit minéral ou organique ou tout mélange de ces produits pulvérulents, non-thermoplastiques dans les conditions opératoires, qui est susceptible d'être compacté. Parmi les produits minéraux, on peut citer le talc, les argiles, en particulier le kaolin, le mica et les pigments minéraux, par exemple les oxydes de titane, de zinc ou de fer. Parmi les produits organiques, on peut citer des poudres végétales telles que l'amidon de riz ou la poudre de soie, et des poudres de polymères non-thermoplastiques, tels que les polyacrylates. Le produit non-thermoplastique organique peut également être constitué par des fibres ou des mélanges de fibres végétales par exemple des fibres de coton.

Les produits non-thermoplastiques définis ci-dessus peuvent être enrobés ou être imprégnés par un fluide d'imprégnation tel qu'une huile, un solvant, l'eau, une solution d'un actif dans l'eau ou dans un solvant. Dans ce cas, on peut obtenir, directement après compactage, un compact absorbant imprégné.

Le produit non-thermoplastique organique peut également être constitué par des microcapsules contenant un fluide, par exemple un fluide d'imprégnation des produits non-thermoplastiques ou un liant. Au cours du processus de compactage, les microcapsules éclatent et le fluide encapsulé va imprégner le reste du produit non-thermoplastique. On obtient ainsi, également, directement après compactage, un produit absorbant imprégné.

Selon l'invention, on utilise, de préférence, un produit non-thermoplastique ayant une granulométrie, homogène ou non, de 2 μ à 200 μ, cette gamme de dimensions correspondant aux longueurs des tronçons de fibres coupées lorsque le produit est une fibre.

Le produit non-thermoplastique peut contenir de 0,5 à 20 % en poids d'un liant facilitant la cohésion des grains de matière pulvérulente au cours du compactage. Le liant est constitué par des produits ou mélange de produits qui sont bien connus parmi lesquels on peut citer le stéarate de zinc, la vaseline, le myristate de nyristyle, l'huile de ricin.

Selon l'invention, on utilise des ultra-sons qui ont avantageusement une fréquence allant de 10 à 100 kHz, et une amplitude de 20 à 60 μm ; puissance envoyée dans le produit en cours de compactage est comprise avantageusement entre 1 et 3 kw par cm3 de compact. L'émission est effectuée pendant un temps généralement compris entre 0,25 s et 3 s ; le temps utilisé croît généralement avec l'épaisseur de la couche du mélange traité.

La pression exercée sur le mélange pulvérulent pendant l'émission des ultra-sons est avantageusement comprise entre 40 et 200 bars. Le piston utilisé, pour comprimer le mélange pulvérulent, est avantageusement constitué par la sonotrode d'un générateur d'ultra-sons.

L'enceinte peut avoir, de façon connue, en section transversale des formes diverses (circulaire, ovale, carrée ou rectangulaire, avec des coins arrondis ou non) et des dimensions quelconques, la surface du piston en contact avec le produit pulvérulent ayant en section transversale la même forme et la même dimension, au jeu nécessaire près pour pouvoir coulisser dans l'enceinte. Le fond de l'enceinte et la surface du piston en contact avec le mélange pulvérulent peuvent être plans ; ils peuvent également avoir indépendamment l'un de l'autre une forme non plane, c'est-à-dire par exemple bombée, munie de motifs décoratifs en relief ou en creux. On peut ainsi obtenir des produits compactés en forme de tuile, de calotte, de pastille, de cachet ou autre.

Selon les différents paramètres du procédé utilisé : pression, puissance et fréquence des ultrasons, temps de maintien des ultra-sons, nature et granulométrie des produits thermoplastiques et non-thermoplastiques, on obtient des produits compactés différents, c'est-à-dire plus ou moins absorbants, plus ou moins délitables et plus ou moins souples ou rigides. Le procédé de la présente demande permet donc d'obtenir une large gamme de produits compactés.

De plus, on a constaté, selon l'invention, que la manière, dont les différents produits pulvérulents sont disposés dans l'enceinte de compactage, a une influence très importante sur la structure du produit compacté.

Selon un premier mode de mise en oeuvre de l'invention, on dispose sur le fond de l'enceinte de compactage, une couche d'un mélange homogène de produit pulvérulent ; par "mélange homogène", on entend ici un mélange dans lequel les différents produits pulvérulents sont répartis de façon régulière. Dans le produit compacté obtenu, le produit thermoplastique forme un réseau régulièrement réparti dans la masse, ledit réseau maintenant dans ses mailles le produit non-thermoplastique. Dans le cas où on cherche à obtenir un produit compacté délitable, le compact obtenu selon ce procédé peut permettre de déliter jusqu'à 15 % en poids de la poudre constitutive du compact.

Selon un second mode de mise en oeuvre du procédé de l'invention, on peut disposer sur le fond de l'enceinte de compactage le mélange pulvérulent sous forme de couches successives dans lesquelles les proportions des produits pulvérulents thermoplastique et non-thermoplastique sont différentes ; de préférence, on dispose sur le fond de l'enceinte de compactage, la couche de mélange pulvérulent ayant la teneur la plus élevée en produit pulvérulent thermoplastique. Plus particulièrement, on dépose sur le fond de l'enceinte deux couches : d'abord une couche de produit thermoplastique, puis une couche de produit non-thermoplastique contenant éventuellement une faible quantité de produit thermoplastique mélangée de façon globalement homogène.

Dans ces conditions, au cours du processus de compactage, le produit thermoplastique déposé sur le fond de l'enceinte remonte partiellement le long des parois latérales de l'enceinte de compactage, une plus grande partie restant dans le fond de l'enceinte; il se produit également une petite pénétration du produit non-thermoplastique dans la couche de produit thermoplastique et de produit thermoplastique dans la couche de produit non-thermoplastique. Le produit compacté se présente donc en deux zones : une zone constituée principalement par le produit thermoplastique, formant en quelque sorte une coupelle ; et une zone disposée à l'intérieur de ladite "coupelle" et constituée principalement de produit non-thermoplastique. Ce second mode de réalisation est particulièrement avantageux, lorsqu'on désire préparer un produit compacté partiellement délitable, tel qu'un compact de produit cosmétique. Il n'est, en effet, plus nécessaire d'effectuer le compactage dans une coupelle, la coupelle étant en quelque sorte formée "in situ" au cours du compactage. Dans ce cas, le compact obtenu peut permettre de déliter jusqu'à 70 % en poids du produit soumis au compactage. Ce second mode de réalisation a également l'avantage de permettre de préparer des produits compactés très minces, ayant une épaisseur de l'ordre d'un millimètre, qui n'ont pas tendance à se briser au cours des différentes manipulations ultérieures.

Selon le procédé de l'invention, après compactage, le produit compacté, quand il est absorbant, peut être chargé par immersion, dans un fluide d'imprégnation.

La présente invention a également pour objet le produit compacté obtenu par le procédé selon l'invention, ce produit constituant un compact absorbant ou partiellement délitable.

Le produit compacté peut, selon l'invention, être un produit absorbant. Dans ce cas, il contient, comme produit non-thermoplastique, au moins un produit absorbant tel que des fibres végétales, les polymères super-absorbants, les argiles ou leurs mélanges.

Le produit compacté absorbant peut être sous forme imprégnable ou sous forme imprégnée d'un fluide d'imprégnation, volatil ou non. Dans ce dernier cas, l'imprégnation peut avoir été effectuée soit en utilisant dans le produit non-thermoplastique des microcapsules contenant le fluide d'imprégnation, soit en immergeant le produit compacté dans le fluide d'imprégnation. On peut ainsi obtenir des produits compactés plus ou moins rigides imprégnés d'huile solaire, de déodorant, de produit détachant ou de parfum. L'utilisateur peut se servir du produit compacté comme applicateur du produit imprégné, un peu à la manière d'un savon. Lorsque le produit d'imprégnation est un produit volatil, on peut utiliser le compact pour provoquer le dégagement du produit volatil, tel que parfum, pesticide, désinfectant ou autre, dans un local.

Le compact obtenu peut également être un produit partiellement délitable, en particulier un compact pour le maquillage du visage. Ce produit compacté obtenu à partir d'une couche de mélange pulvérulent homogène est constitué par un réseau thermoplastique régulièrement réparti maintenant dans ses mailles le produit non-thermoplastique. Dans ce cas, le produit compacté peut contenir jusqu'à 15 % en poids de produit délitable. Le compact obtenu peut également être un produit partiellement délitable comportant deux zones : une zone formant coupelle constituée en majeure partie de produit thermoplastique et une zone localisée à l'intérieur de la précédente et constituée en majeure partie de produit non-thermoplastique ; un tel compact est obtenu par compactage d'un mélange en couches successives ; dans ce cas, le compact peut contenir jusqu'à 70 % en poids de produit délitable. Un tel compact peut avoir une épaisseur de l'ordre du millimètre sans risque de se briser au cours des manipulations, qui suivent le compactage.

On peut également réaliser le compactage directement sur un support en matière thermofusible permettant l'utilisation du compact. Dans ce cas, on prévoit que le support comporte des moyens pour solidariser le compact avec lui. On peut notamment prévoir de réaliser le compactage par le procédé selon l'invention sur un support rigide ou flexible floqué ; dans ce cas, les poils du floc sont insérés dans le compact après réalisation de celui-ci et maintiennent le compact sur le support. Si l'on réalise de la sorte un compact de maquillage, en réalisant le compactage uniquement dans la zone centrale du support floqué, on peut utiliser le support pour une application directe du produit de maquillage, le compact déposant alors sur la peau la poudre délitable qu'il renferme en surface et les poils périphériques du support floqué qui ne sont pas recouverts par le compact permettant un estompage de la poudre de maquillage déposée sur la peau. Un tel support peut d'ailleurs comporter, sur celle de ses faces qui ne porte pas le compact, une autre surface floquée permettant de finir l'estompage du maquillage. Le support de compactage peut être réalisé également par une feuille en matière non-tissée comportant une phase en matière thermoplastique telle qu'une feuille non-tissée formée par 70 % en poids par des fibres de coton et par 30 % de fibres de polypropylène.

Les exemples donnés ci-après, à titre purement illustratif et non limitatif, permettront de mieux comprendre l'invention.

Dans tous les exemples donnés ci-après, on utilise une enceinte de compactage cylindrique ayant une section droite circulaire de 30 mm de diamètre, dans laquelle coulisse une sonotrode cylindrique de même diamètre, au jeu près, reliée à un générateur d'ultra-sons. Les ultra-sons ont une fréquence de 30 kHz ; l'émission s'effectue à une puissance de 2 kw. Le temps d'application de l'émission est de 2 s. La pression de compactage exercée sur la sonotrode est de 100 bars : elle est maintenue pendant 2,5 s.

EXEMPLE 1 - Préparation d'une pastille absorbante plate non imprégnée

On dépose sur le fond de l'enceinte de compactage une couche d'un mélange pulvérulent homogène ayant la composition suivante (en % en poids) :

```
- Produit thermoplastique :
    . Polyamide vendu sous la dénomination
      commerciale "Nylon 6.6" ayant une
      granulométrie comprise entre 10 µm et
      300 µm.............................     60 %
- Produit non thermoplastique :
    . Fibres de coton
      (longueur : de 40 µ à 1 mm) ..........     2 %
    . Poudre de polyacrylate vendue sous la
      dénomination commerciale "SUMIKAGEL"
      ayant une granulométrie comprise
      entre 10 µm et 300 µm ................    30 %
    . Talc ayant une granolumétrie comprise
      entre 5 µm et 200 µm ................     8 %
```

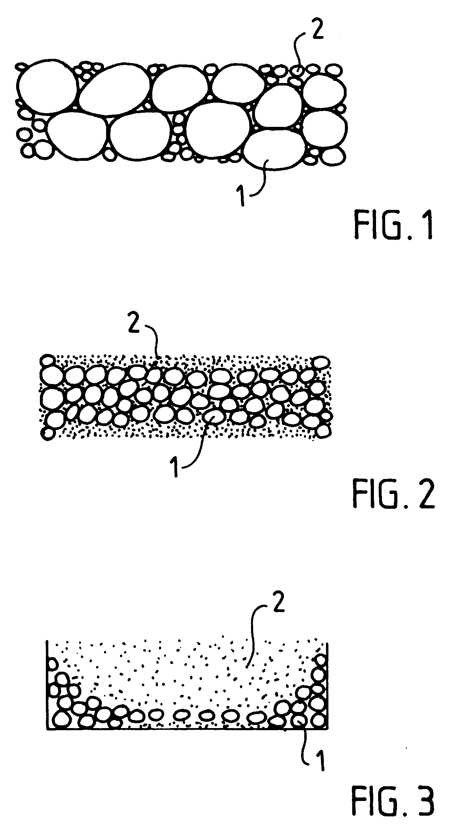

On obtient une pastille absorbante non imprégnée ayant en coupe la structure schématisée sur la figure 1, où la référence 1 désigne les grains de produit thermoplastique et la référence 2 les grains de produit non-thermoplastique. Cette pastille peut facilement être imprégnée, grâce au super-absorbant acrylique, d'une solution aqueuse de principe actif.

EXEMPLE 2 - Préparation d'une pastille compactée imprégnée

On opère comme dans l'exemple 1, mais on utilise des fibres de coton ayant une longueur de 500 µm imprégnées de parfum à raison de 5 % en poids de parfum par rapport au poids de fibres ; le super-absorbant est imprégné d'un principe actif constitué par une solution aqueuse et d'alcool à raison de 300 % en poids par rapport au poids de super-absorbant. Dans la formulation de la composition de l'exemple 1, on remplace, en conservant les proportions, les fibres et le polyacrylate non imprégnés par les mêmes constituants pré-imprégnés.

La pastille obtenue après compactage peut être utilisée directement par application sur la peau par l'utilisateur.

EXEMPLE 3 - Préparation d'un compact de poudre cosmétique rigide

On dépose sur le fond de l'enceinte une couche de 5 cm d'un mélange pulvérulent homogène ayant la composition suivante (en % en poids) :

- Produit thermoplastique :

    . Cire de polyéthylène vendue sous la dénomination commerciale "AKUMIST"

      par la société "ALLIED CHEMICAL"

      de granulométrie comprise

      entre 2 et 300 µm .....................   45 %

- Produit non-thermoplastique :

    . Poudre de soie pigmentée enrobée de 5 % en poids (par rapport au poids de la poudre de soie non enrobée) d'un liant constitué par du myristate de

      myristyle et ayant une granulométrie

      de 200 µm ...........................   55 %

On a constaté que, dans la pastille compactée obtenue, la structure est celle schématisée sur la figure 2, où la référence 1 indique les grains de produit thermoplastique et la référence 2 les grains de produit non-thermoplastique. La zone formant coupelle est à l'intérieur du compact et est délimitée par deux surfaces de concavité opposée.

La pastille obtenue est rigide : elle peut être mise en oeuvre par délitage au moyen d'un pinceau, la poudre ainsi reprise constituant au plus 15 % du poids total de la pastille.

EXEMPLE 4 - Préparation d'un compact de poudre cosmétique comportant une coupelle souple formée "in-situ"

On a déposé sur le fond de l'enceinte de compactage, en premier lieu une couche, de 5 mm d'épaisseur, d'un produit thermoplastique constitué par une poudre de polyéthylène basse densité de marque "ENKA FRAGRANCE CARRIER", commercialisée par la société "ENKA AG.", puis en second lieu, une couche de 5 mm d'épaisseur d'un produit non-thermoplastique constitué d'une poudre de soie pigmentée enrobée de 5 % en poids (par rapport au poids de la poudre de soie non enrobée) d'un liant constitué par du myristate de myristyle. Le polyéthylène représente 15 % en poids du mélange et a une granulométrie de 50 µm. La poudre enrobée a une granulométrie de 200 µm.

Après compactage, on obtient une pastille ayant la structure schématisée sur la figure 3 où la référence 1 indique les grains de produit thermoplastique et la référence 2 les grains de produit non-thermoplastique. La zone formant coupelle est souple et disposée à l'extérieur. Le compact obtenu a une épaisseur de 2 mm et on peut le manipuler sans le briser. On a constaté qu'environ 50 % en poids de la poudre est délitable à l'aide d'un pinceau pour être utilisée.

EXEMPLE 5 - Préparation d'une pastille compactée imprégnée

On a déposé sur le fond de l'enceinte une couche d'un mélange homogène ayant la composition suivante (en % en poids) :

```
- Produit thermoplastique :
    . Polyamide vendu sous le dénomination
      commerciale "Nylon 6.6" ayant une
      granulométrie de 70 µm ..................... 50 %
- Produit non thermoplastique :
    . Talc ayant une granulométrie de 150 µm ..... 45 %
    . Microcapsules de parfum contenant
      un parfum et ayant une granulométrie
      de 80 µm .................................. 5 %
```

La majorité des microcapsules éclate au cours du compactage et le parfum va imprégner le talc. Le parfum relargue ensuite lentement vers l'extérieur de la pastille compactée. La pastille obtenue peut être utilisée pour parfumer un local.

EXEMPLE 6 - Préparation d'un compact de poudre cosmétique flexible

On reproduit l'exemple 3 en remplaçant la cire de polyéthylène vendue sous la dénomination commerciale "AKUMIST" par une poudre de polyéthylène basse densité vendue sous la dénomination commerciale "ACCUREL" par la société "ENKA", ladite poudre étant constituée de particules polymères microporeuses.

On a constaté que la pastille compactée obtenue était flexible et pouvait être mise en oeuvre par délitage au moyen d'un pinceau, la poudre ainsi reprise pouvant constituer jusqu'à environ 10 % du poids total de la pastille.

EXEMPLE 7 - Préparation d'une pastille absorbante plate non imprégnée

On réalise une pastille compactée comme indiqué dans l'exemple 1 en remplaçant simplement le polyamide vendu sous la dénomination commerciale "Nylon 6.6" par la même quantité de poudre de polyéthylène basse densité, vendue sous la dénomination commerciale "ACCUREL" et déjà utilisée à l'exemple 6.

On obtient une pastille absorbante qui peut être facilement imprégnée, grâce au super absorbant acrylique, d'une solution aqueuse de principe actif.

EXEMPLE 8 - Préparation d'une pastille compactée imprégnée

On réalise une pastille compactée imprégnée comme indiqué dans l'exemple 5 en remplaçant le polyamide vendu sous la dénomination commerciale "Nylon 6.6" par la poudre de polyéthylène basse densité, vendue sous la dénomination commerciale "ACCUREL" déjà mentionnée à l'exemple 6.

On obtient une pastille compactée imprégnée ayant des caractéristiques analogues à celles de l'exemple 5.

**Revendications**

1.  Procédé de compactage d'un mélange pulvérulent permettant d'obtenir un produit compacté absorbant ou partiellement délitable, dans lequel on dépose une quantité de mélange pulvérulent au fond d'une enceinte et on fait coulisser dans l'enceinte un piston qui exerce une pression de compactage sur le mélange pulvérulent lequel contient au moins un produit tnermoplastique , le reste étant constitué par au moins un produit non-thermoplastique, caractérisé par le fait que le mélange pulvérulent contient 5 à 80 % en poids de produit thermoplastique, et que, pendant que l'on exerce la pression, on soumet le mélange pulvérulent à l'action d'ultra-sons.

2.  Procédé selon la revendication 1, caractérisé par le fait que le produit thermoplastique a une granulométrie comprise entre 5 et 500 $\mu$.

7

3. Procédé selon l'une des revendications 1 ou 2, caractérisé par le fait que le produit non-thermoplastique a une granulométrie comprise entre 2 et 400 μ.

4. Procédé selon l'une des revendications 1 à 3, caractérisé par le fait que le produit non-thermoplastique contient de 0,5 à 20 % en poids d'un liant.

5. Procédé selon l'une des revendications 1 à 4, caractérisé par le fait qu'on utilise des ultrasons ayant une fréquence allant de 10 à 100 kHz et une amplitude de 20 à 60 μm, la puissance envoyée dans le produit au cours de compactage étant comprise entre 1 et 3 kw par cm3 de compact.

6. Procédé selon l'une des revendications 1 à 5, caractérisé par le fait que l'émission est effectuée pendant un temps compris entre 0,25 et 3 s.

7. Procédé selon l'une des revendications 1 à 6, caractérisé par le fait que la pression exercée sur le mélange pulvérulent pendant l'émission des ultra-sons est comprise entre 40 et 200 bars.

8. Procédé selon l'une des revendications 1 à 7, caractérisé par le fait que le piston utilisé pour comprimer le mélange pulvérulent est constitué par la sonotrode d'un générateur d'ultra-sons.

9. Procédé selon l'une des revendications 1 à 8, caractérisé par le fait qu'on dispose sur le fond de l'enceinte de compactage une couche d'un mélange homogène de produit pulvérulent.

10. Procédé selon l'une des revendications 1 à 8, caractérisé par le fait qu'on dispose, sur le fond de l'enceinte de compactage, le mélange pulvérulent sous forme de couches successives dans lesquelles les proportions des produits pulvérulents thermoplastique et non-thermoplastique sont différentes, la couche de mélange pulvérulent ayant la teneur la plus élevée en produit pulvérulent thermoplastique étant disposée sur le fond de l'enceinte de compactage.

11. Procédé selon la revendication 10, caractérisé par le fait qu'on dispose sur le fond de l'enceinte deux couches, à savoir d'abord une couche de produit thermoplastique, puis une couche de produit non-thermoplastique.

12. Procédé selon l'une des revendications 1 à 11, caractérisé par le fait qu'après compactage, le produit compacté est chargé par immersion dans un fluide d'imprégnation.

13. Produit compacté obtenu par le procédé de l'une des revendications 1 à 12, constituant un compact absorbant ou partiellement délitable.

14. Produit selon la revendication 13 constituant un compact absorbant, caractérisé par le fait qu'il contient, comme produit(s) non-thermoplastique(s), des fibres végétales et/ou un acrylate et/ou un polyacrylate super-absorbant et/ou une argile.

15. Produit selon la revendication 14, caractérisé par le fait qu'il est chargé d'un fluide d'imprégnation, volatil ou non.

16. Produit selon la revendication 15, caractérisé par le fait que le fluide d'imprégnation est un parfum.

17. Produit selon la revendication 13, constituant un compact partiellement délitable, caractérisé par le fait qu'il comporte un réseau de produit thermoplastique régulièrement réparti dans la masse, ledit réseau maintenant le produit non-thermoplastique.

18. Produit selon la revendication 17, caractérisé par le fait qu'il contient jusqu'à 15 % en poids de produit délitable.

19. Produit selon la revendication 13, constituant un compact partiellement délitable, caractérisé par le fait qu'il comporte une zone formant coupelle constituée en majeure partie de produit thermoplastique et une zone disposée à l'intérieur de ladite coupelle constituée en majeure partie de produit non-thermoplastique.

**20.** Produit selon la revendication 19, caractérisé par le fait qu'il contient jusqu'à 70 % en poids de produit délitable.

**21.** Produit selon l'une des revendications 13 à 20, caractérisé par le fait qu'il est porté par un support sur lequel est réalisé le compactage, ledit support comportant en surface des éléments qui assurent la liaison avec le produit compacté.

**22.** Produit selon la revendication 21, caractérisé par le fait que le support de compactage est floqué au moins sur celle de ses faces où est réalisé le compactage.

**23.** Produit selon la revendication 21, caractérisé par le fait que le support de compactage est une feuille comportant une phase thermoplastique.

**Claims**

**1.** Process for compacting a pulverulent mixture making it possible to obtain an absorbent or partially friable compacted product, in which a quantity of pulverulent mixture is deposited at the bottom of an enclosure and a piston is caused to slide in the enclosure, exerting a compacting pressure on the pulverulent mixture which contains at least one thermoplastic product, the remainder consisting of at least one nonthermoplastic product, characterized in that the pulverulent mixture contains 5 to 80 % by weight of thermoplastic product and that the pulverulent mixture is subjected to the action of ultrasound while the pressure is exerted.

**2.** Process according to Claim 1, characterized in that the thermoplastic product has a particle size of between 5 and 500 $\mu$.

**3.** Process according to either of Claims 1 and 2, characterized in that the nonthermoplastic product has a particle size of between 2 and 400 $\mu$.

**4.** Process according to one of Claims 1 to 3, characterized in that the nonthermoplastic product contains from 0.5 to 20 % by weight of a binder.

**5.** Process according to one of Claims 1 to 4, characterized in that ultrasound which has a frequency ranging from 10 to 100 kHz and an amplitude of 20 to 60 $\mu$m is employed, the power transmitted into the product being compacted being between 1 and 3 kW per cm$^3$ of compact.

**6.** Process according to one of Claims 1 to 5, characterized in that the transmission is performed for a period of between 0.25 and 3 s.

**7.** Process according to one of Claims 1 to 6, characterized in that the pressure exerted on the pulverulent mixture during the transmission of the ultrasound is between 40 and 200 bars.

**8.** Process according to one of Claims 1 to 7, characterized in that the piston employed for compressing the pulverulent mixture consists of the sonotrode of an ultrasonic generator.

**9.** Process according to one of Claims 1 to 8, characterized in that a layer of a homogeneous mixture of pulverulent product is placed on the bottom of the compacting enclosure.

**10.** Process according to one of Claims 1 to 8, characterized in that on the bottom of the compacting enclosure the pulverulent mixture is arranged in the form of successive layers in which the proportions of the thermoplastic and nonthermoplastic pulverulent products are different, the layer of pulverulent mixture having the highest content of thermoplastic pulverulent product being placed on the bottom of the compacting enclosure.

**11.** Process according to Claim 10, characterized in that two layers are placed on the bottom of the enclosure, namely first of all a layer of thermoplastic product and then a layer of nonthermoplastic product.

9

12. Process according to one of Claims 1 to 11, characterized in that after compacting the compacted product is filled by immersion in an impregnating fluid.

13. Compacted product obtained by the process of one of Claims 1 to 12, forming an absorbent or partially friable compact.

14. Product according to Claim 13, forming an absorbent compact, characterized in that it contains vegetable fibres and/or an acrylate and/or a superabsorbent polyacrylate and/or a clay as nonthermoplastic product(s).

15. Product according to Claim 14, characterized in that it is filled with a volatile or nonvolatile impregnating fluid.

16. Product according to Claim 15, characterized in that the impregnating fluid is a perfume.

17. Product according to Claim 13, forming a partially friable compact, characterized in that it comprises a lattice of thermoplastic product uniformly distributed in the bulk, the said lattice keeping the product nonthermoplastic.

18. Product according to Claim 17, characterized in that it contains up to 15 % by weight of friable product.

19. Product according to Claim 13, forming a partially friable compact, characterized in that it comprises a zone forming a small dish consisting for the most part of thermoplastic product and a zone arranged inside the said small dish, consisting for the most part of non-thermoplastic product.

20. Product according to Claim 19, characterized in that it contains up to 70 % by weight of friable product.

21. Product according to one of Claims 13 to 20, characterized in that it is carried by a support on which the compacting is carried out, the said support comprising, at the surface, components which ensure the bonding with the compacted product.

22. Product according to Claim 21, characterized in that the compacting support is flocked at least on that of its faces where the compacting is carried out.

23. Product according to Claim 21, characterized in that the compacting support is a sheet comprising a thermoplastic phase.

**Patentansprüche**

1. Verfahren zum Kompaktieren eines pulverförmigen Gemisches, das es ermöglicht, ein kompaktiertes saugfähiges oder teilweise abbröckelbares Produkt zu erzeugen, bei dem man eine Menge des pulverförmigen Gemisches auf dem Boden einer Einfassung anordnet und einen Kolben in der Einfassung verschiebt, der einen Kompaktierungsdruck auf das pulverförmige Gemisch ausübt, das zumindest ein thermoplastisches Produkt enthält, wobei der Rest von mindestens einem nicht-thermoplastischen Produkt gebildet wird,
**dadurch gekennzeichnet**,
daß das pulverförmige Gemisch 5 bis 80 Gewichtsprozent des thermoplastischen Produkts enthält und, daß man das pulverförmige Gemisch, während man den Druck ausübt, der Einwirkung von Ultraschall aussetzt.

2. Verfahren nach Anspruch 1, dadurch gekennzeichnet, daß das thermoplastische Produkt eine Korngröße zwischen 5 und 500 $\mu$ aufweist.

3. Verfahren nach einem der Ansprüche 1 oder 2, dadurch gekennzeichnet, daß das nicht-thermoplastische Produkt eine Korngröße zwischen 2 und 400 $\mu$m aufweist.

4. Verfahren nach einem der Ansprüche 1 bis 3, dadurch gekennzeichnet, daß das nicht-thermoplastische Produkt 0,5 bis 20 Gewichtsprozent eines Bindemittels enthält.

10

5. Verfahren nach einem der Ansprüche 1 bis 4, dadurch gekennzeichnet, daß man Ultraschall mit einer Frequenz von 10 bis 100 kHz und einer Amplitude von 20 bis 60 $\mu$m anwendet, wobei die dem Produkt während des Kompaktierens zugeführte Leistung zwischen 1 und 3 kW/cm$^3$ des Preßlings beträgt.

6. Verfahren nach einem der Ansprüche 1 bis 5, dadurch gekennzeichnet, daß die Emission während einer Zeitdauer zwischen 0,25 und 3 s erfolgt.

7. Verfahren nach einem der Ansprüche 1 bis 6, dadurch gekennzeichnet, daß der während der Ultraschall-Emission auf das pulverförmige Gemisch ausgeübte Druck zwischen 40 und 200 bar beträgt.

8. Verfahren nach einem der Ansprüche 1 bis 7, dadurch gekennzeichnet, daß der zum Komprimieren des pulverförmigen Gemisches verwendete Kolben von der Sonotrode eines Ultraschallerzeugers gebildet wird.

9. Verfahren nach einem der Ansprüche 1 bis 8, dadurch gekennzeichnet, daß man auf dem Boden der Kompaktiereinfassung eine Schicht einer homogenen Mischung des pulverförmigen Produktes anordnet.

10. Verfahren nach einem der Ansprüche 1 bis 8, dadurch gekennzeichnet, daß man das pulverförmige Gemisch in Form von aufeinanderfolgenden Schichten, in denen die Mischungsverhältnisse der thermoplastischen und nicht-thermoplastischen pulverförmigen Produkte unterschiedlich sind, auf dem Boden der Kompaktiereinfassung anordnet, wobei man die Schicht des pulverförmigen Produktes mit dem höchsten Gehalt des pulverförmigen thermoplastischen Produktes auf dem Boden der Kompaktiereinfassung anordnet.

11. Verfahren nach Anspruch 10, dadurch gekennzeichnet, daß man auf dem Boden der Kompaktiereinfassung zwei Schichten anordnet, und zwar zunächst eine Schicht des thermoplastischen Produktes und dann eine Schicht des nicht-thermoplastischen Produktes.

12. Verfahren nach einem der Ansprüche 1 bis 11, dadurch gekennzeichnet, daß das kompaktierte Produkt nach dem Kompaktieren durch Eintauchen in eine Imprägnierflüssigkeit beladen wird.

13. Kompaktiertes Produkt, welches durch das Verfahren nach einem der Ansprüche 1 bis 12 erhalten wird und einen saugfähigen oder teilweise abbröckelbaren Preßkörper bildet.

14. Produkt nach Anspruch 13, das einen saugfähigen Preßkörper bildet, dadurch gekennzeichnet, daß es als nichtthermoplastisches Produkt (nicht-thermoplastische Produkte) Pflanzenfasern und/oder ein Acrylat und/oder ein supersaugfähiges Polyacrylat und/oder Ton enthält.

15. Produkt nach Anspruch 14, dadurch gekennzeichnet, daß es mit einer flüchtigen oder nicht flüchtigen Imprägnierflüssigkeit beladen ist.

16. Produkt nach Anspruch 15, dadurch gekennzeichnet, daß die Imprägnierflüssigkeit ein Parfüm ist.

17. Produkt nach Anspruch 13, das einen teilweise abbröckelbaren Preßkörper bildet, dadurch gekennzeichnet, daß es ein gleichmäßig in der Masse verteiltes Netz des thermoplastischen Produkts umfaßt, wobei das Netz das nicht-thermoplastische Produkt zusammenhält.

18. Produkt nach Anspruch 17, dadurch gekennzeichnet, daß es bis zu 15 Gewichtsprozent des abbröckelbaren Produktes enthält.

19. Produkt nach Anspruch 13, das einen teilweise abbröckelbaren Preßkörper bildet, dadurch gekennzeichnet, daß es einen von einem Großteil des thermoplastischen Produkts gebildeten tiegelförmigen Bereich und einen von einem Großteil des nichtthermoplastischen Produkts gebildeten, im Inneren des Tiegels angeordneten Bereich umfaßt.

**20.** Produkt nach Anspruch 19, dadurch gekennzeichnet, daß es bis zu 70 Gewichtsprozent des abbröckelbaren Produkts enthält.

**21.** Produkt nach einem der Ansprüche 13 bis 20, dadurch gekennzeichnet, daß es durch einen Träger getragen ist, auf dem die Kompaktierung durchgeführt wird, wobei der Träger auf der Oberfläche Elemente aufweist, welche die Verbindung mit dem kompaktierten Produkt gewährleisten.

**22.** Produkt nach Anspruch 21, dadurch gekennzeichnet, daß der Kompaktierungsträger zumindest auf derjenigen seiner Seiten, wo die Kompaktierung durchgeführt wird, beflockt ist.

**23.** Produkt nach Anspruch 21, dadurch gekennzeichnet, daß der Kompaktierungsträger eine Folie ist, die eine thermoplastische Phase aufweist.

FIG. 1

FIG. 2

FIG. 3